# EUROPEAN PATENT APPLICATION

(11) **EP 1 767 652 A1**
(43) Date of publication of application: **28.03.2007**
(21) Application number: 05743891.3
(22) Date of filing: 30.05.2005
(51) Int. Cl.: C12Q 1/68, C12N 15/09, G01N 33/50

(54) **GENE MARKER AND UTILIZATION OF THE SAME**

(30) Priority: 02.06.2004 JP 2004165013; 13.01.2005 JP 2005006727
(71) Applicant: Keio University, Tokyo 108-8345 (JP)
(72) Inventor: TANIGAWARA, Yusuke Keio University School Medicine, Shinjuku-ku Tokyo 1608582 (JP); IKETANI, Osamu Keio University School of Medicine, Shinjuku-ku Tokyo 1608582 (JP); MIHARA, Kiyoshi Keio University School of Medicine, Shinjuku-ku Tokyo 1608582 (JP)
(74) Representative: Jones, Helen M.M.
(86) International application number: PCT/JP2005/009871
(87) International publication number: WO 2005/121366

(57) **Abstract**

[PROBLEMS]

To provide a gene marker which enables diagnosis of rejection, evaluation of the efficacy of an immunosuppressive agent, and determination of the presence or absence of immunological tolerance; methods that can be performed in a quick, simple, and convenient manner by using a gene marker as an indicator for diagnosing rejection, evaluating the efficacy of an immunosuppressive agent, identifying an immunosuppressive agent, selecting an immunosuppressive agent, determining the dose of an immunosuppressive agent, and judging the presence or absence of immunological tolerance; and a kit.

[MEANS FOR SOLVING PROBLEMS]

Immune-related genes whose expression levels were increased by 1.5-fold or more because of rejection and whose expression levels were decreased by 1.5-fold or more because of immunosuppressive agents have been identified as gene markers. By using the expression level of one of these gene markers as an indicator, it becomes possible to diagnose rejection, evaluate the efficacy of an immunosuppressive agent, and judge the presence or absence of immunological tolerance in a quick, simple, and convenient manner.

SEQUENCE LISTING

<110> KEIO UNIVERSITY

<120> GENE MARKER AND USE THEREOF

<130> HMJ04315EP

<140> EP05743891.3
<141> 2005-05-30

<150> JP 2004/165013
<151> 2004-06-02

<150> JP 2005/6727
<151> 2005-01-13

<160> 15

<170> PatentIn version 3.1

<210> 1
<211> 17
<212> DNA
<213> Artificial Sequence

<220>
<223> Inventor: Tanigawara, Yusuke
Inventor: Iketani, Osamu
Inventor: Mihara, Kiyoshi

<220>
<223> Primer

<400> 1
gccgtcttcc cctccat

17

<210> 2
<211> 22
<212> DNA
<213> Artificial Sequence

<220>
<223> Primer

<400> 2
aggagtcctt ctgacccata cc

22

<210> 3
<211> 24
<212> DNA
<213> Artificial Sequence

<220>
<223> Primer

<400> 3
caccactgat ctgtacaact tgca

24

<210> 4
<211> 25
<212> DNA
<213> Artificial Sequence

<220>
<223> Primer

<400> 4
cactcagact gttcaaagag cttca

25

<210> 5
<211> 21
<212> DNA
<213> Artificial Sequence

<220>
<223> Primer

<400> 5
agaagtccac accgggacaa c

21

<210> 6
<211> 21
<212> DNA
<213> Artificial Sequence

<220>
<223> Primer

<400> 6
tgtcaaacac gcggttcact a

21

<210> 7
<211> 18
<212> DNA
<213> Artificial Sequence

<220>
<223> Primer

<400> 7
ggtctactcg ggcatccg

18

<210> 8
<211> 19
<212> DNA
<213> Artificial Sequence

<220>
<223> Primer

<400> 8
ggtccttctc cacgtgctt

19

<210> 9
<211> 22
<212> DNA
<213> Artificial Sequence

<220>
<223> Primer

<400> 9
ctggtggtga caagcacatt tg

22

<210> 10
<211> 24
<212> DNA
<213> Artificial Sequence

<220>
<223> Primer

<400> 10
gtatgcccga gttctttcat catg

24

<210> 11
<211> 18
<212> DNA
<213> Artificial Sequence

<220>
<223> Probe

<400> 11
ccatcacacc ctggtgcc

18

<210> 12
<211> 18
<212> DNA
<213> Artificial Sequence

<220>
<223> Probe

<400> 12
acctctgaag ctgcaaca

18

<210> 13
<211> 21
<212> DNA
<213> Artificial Sequence

<220>
<223> Probe

<400> 13
ggctgctccc gctgacactc g

21

<210> 14
<211> 16
<212> DNA
<213> Artificial Sequence

<220>
<223> Probe

<400> 14
atggccaccg gcaagt

16

<210> 15
<211> 20
<212> DNA
<213> Artificial Sequence

<220>
<223> Probe

<400> 15
cttcagagtc tgcccattgc

20

## Description

### CROSS REFERENCE TO RELATED APPLICATIONS

This application claims the benefit of priority to Japanese Patent Application No. 2004-165013, filed on June 2, 2004, and Japanese Patent Application No. 2005-6727, filed on January 13, 2005, which are incorporated herein by reference.

### TECHNICAL FIELD

The present invention relates to gene markers and methods for diagnosing rejection, methods for judging the presence or absence of immunological tolerance, methods for evaluating the efficacy of immunosuppressive agents, methods for identifying immunosuppressive agents, methods for selecting immunosuppressive agents, methods for determining the doses of immunosuppressive agents, kits, and methods for screening for immunosuppressive agents or immunological tolerance-inducing agents.

### BACKGROUND ART

Organ transplantation is commonly performed as a therapeutic strategy for saving lives of patients with severe organ failure and for improving their QOL (quality of life). However, because of rejection, which is a serious complication, a transplanted organ is sometimes lost, so that the therapy turns out to be unsuccessful. For this reason, it is expected to develop methods for quickly diagnosing rejection, agents for controlling rejection (immunosuppressive agents), and the like with a view to taking early preventive measures against rejection.

As a method for diagnosing rejection after organ transplantation, tissue biopsies (needle biopsies) under local anesthesia is regarded as the most reliable. However, since this method is invasive and imposes a great burden upon patients, repeated treatment is impossible. Moreover, since performing a need biopsy requires skilled technique and adequate equipment, it cannot be performed easily. Currently, needle biopsies have been replaced with blood tests (for example, methods for measuring a change in concentration of white blood cells (WBC), CRP (C-reactive protein), organopathy markers (AST (aspartate aminotransferase; GOT) or ALT (alanine aminotransferase; GPT)), bilirubin, gamma GTP (guanosine triphosphate), creatinine, or blood urea nitrogen (BUN) in blood), Doppler ultrasonography (observation of blood stream), etc. However, all these are only auxiliary methods; none of these alone can diagnose the presence or absence of rejection. Therefore, there is a demand for the development of biomarkers for diagnosing the presence or absence of rejection in place of needle biopsies.

Biomarkers for evaluating rejection of transplanted tissues previously identified include perforin, granzyme B, Fas ligand, etc. (refer to National Publication of International Patent Application No. 2001-517459).

After transplantation, to suppress the above-described rejection, an immunosuppressive agent is administered to the patient who has undergone transplantation. Immunosuppressive agents currently in use include cyclosporine (refer to e.g., Japanese patent No. 3382656), FK506 (tacrolimus : refer to, e.g., U.S. Patent No. 4894366), etc. These immunosuppressive agents cause adverse side effects such as infection because of overdosing; therefore it is absolutely essential to precisely determine the usage (modes of administration) and dosage (doses and dosing intervals) of each immunosuppressive agent.

The doses of immunosuppressive agents are determined by monitoring whether the blood concentrations of the drugs are within the effective blood concentrations of the drugs (therapeutic drug monitoring: TDM) However, such effective blood concentrations are determined empirically, not based on scientific evidence, and therefore do not necessarily serve as indicators of the efficacy.

On the other hand, there are a small number of patients whose rejection is reduced to the level where they no longer need an immunosuppressive agent and who can stop taking the immunosuppressive agent. Tolerance induction like this is considered to occur partly because immunosuppressive agents have tolerance-inducing activity. Thus, those patients who have acquired immunological tolerance can stop taking an immunosuppressive agent; they can be released from adverse side effects, such as infection, the risk of malignant tumors, or organopathy, associated with long-term administration of an immunosuppressive agent. Accordingly, in recent years, development of methods have bee attempted for artificially inducing immunological tolerance by, for example, inhibiting the co-stimulatory molecule CD28 expressed in naive T cells by administering an antibody or a recombinant protein. Alternatively, there are other attempts to induce immunological tolerance by being creative with mode of administration or by administering a plurality of immunosuppressive agents (in which case, immunosuppressive agent(s) are serving as tolerance-inducting agents as well).

However, whether or not immunological tolerance has been acquired cannot be correctly determined, because there is no scientific indicator of immunological tolerance; disadvantageously, discontinuation of administration of immunosuppressive agent (s) in judging the presence or absence of acquisition of immunological tolerance has always entailed the danger of causing rejection. Therefore, there is a demand for the development of methods that make it possible to judge the presence or absence of immunological tolerance in a quick, simple, and convenient manner.

### DISCLOSURE OF THE INVENTION

### PROBLEMS TO BE SOLVED BY THE INVENTION

The present invention has been made in consideration of the above-mentioned problem. The object of the present invention is to provide gene markers that enables diagnosis of rejection, evaluation of drug efficacy, and judgment of the presence or absence of immunological tolerance; methods that can be performed in a quick, simple, and convenient manner using the gene markers as indicators for diagnosing rejection, for judging the presence or absence of immunological tolerance, for evaluating the efficacy of an immunosuppressive agent, for identifying an immunosuppressive agent, for selecting an immunosuppressive agent, and for determining the dose of an immunosuppressive agent; kits; and methods for screening for an immunosuppressive agent or an immunological tolerance-inducing agent.

### MEANS FOR SOLVING THE PROBLEMS

To solve the above-mentioned problem, the inventors of the present application have identified immune-related genes (IRF1, PSMB9, NOS2A, PIM1, TAP1, CTSS, etc.) whose expression levels increased by 1.5-fold or more because of rejection decreased by 1.5-fold or more because of an immunosuppressive agent, by analyzing gene expression patterns in the peripheral blood of rejection model rats using microarrays at early stages of acute rejection and at time of administration of an immunosuppressive agent. Further, by examining the expression levels of the above-mentioned genes relative to the amount of the immunosuppressive agent used and the blood concentration of the immunosuppressive agent that had been administered to the rejection model rats, the present inventors clarified that there is a correlation between the dose or the blood concentration of an immunosuppressive agent and the expression levels of the above-mentioned genes. Thus, the present invention has been accomplished.

Namely, the gene marker according to the present invention serves as an indicator of rejection, and the gene marker is a gene-related substance related to a gene selected from the group consisting of IRF1, PSMB9, NOS2A, PIM1, TAP1, and CTSS. The gene marker serves as an indicator of rejection due to transplantation of a tissue or an organ, such as a heart, a kidney, a liver, etc .

Further, the gene marker according to the present invention serves as an indicator of immunological tolerance, and the gene marker is a gene-related substance related to a gene selected from the group consisting of IRF1, PSMB9, NOS2A, PIM1, TAP1, and CTSS. For example, the gene marker serves as an indicator of immunological tolerance to transplantation of a tissue or an organ, such as a heart, a kidney, a liver, etc.

Further, the gene marker according to the present invention serves as an indicator of the efficacy of an immunosuppressive agent, and the gene marker is a gene-related substance related to a gene selected from the group consisting of IRF1, PSMB9, NOS2A, PTM1, TAP1, and CTSS. For example, the gene marker serves as an indicator of the efficacy of an immunosuppressive agent for transplantation of a tissue or an organ, such as a heart, a kidney, a liver, etc.

The method for diagnosing rejection according to the present invention diagnoses rejection by measuring a change in the expression level of a gene marker in blood drawn from a vertebrate other than a human, and the gene marker to be used is a gene-related substance related to a gene selected from the group consisting of IRF1, PSMB9, NOS2A, PIM1, TAP1, and CTSS. For example, the method for diagnosing rejection according to the present invention can examine the presence or absence of rejection due to transplantation of a tissue or an organ, such as a heart, a kidney, a liver, etc.

Further, the kit for diagnosing rejection according to the present invention includes a primer or an antibody for measuring a change in the expression level of a gene marker in blood, and the gene marker to be used is a gene-related substance related to a gene selected from the group consisting of IRF1, PSMB9, NOS2A, PIM1, TAP1, and CTSS. For example, the kit for diagnosing rejection according to the present invention can examine the presence or absence of rejection due to transplantation of a tissue or an organ, such as a heart, a kidney, a liver, etc.

The method for judging the presence or absence of immunological tolerance according to the present invention judges the presence or absence of immunological tolerance by measuring a change in the expression level of a gene marker in blood drawn from a vertebrate other than a human into which a tissue or an organ has been transplanted. The gene marker to be used is a gene-related substance related to a gene selected from the group consisting of IRF1, PSMB9, NOS2A, PIM1, TAP1, and CTSS. The aforementioned tissue or organ is, for example, a heart, a kidney, a liver, etc.

Further, the method for judging the presence or absence of immunological tolerance according to the present invention includes the steps of: monitoring the expression level of a gene marker in blood drawn from a vertebrate into which a tissue or an organ has been transplanted while reducing the dose of an immunosuppressive agent administered to the vertebrate after the transplantation; and judging that the immunological tolerance has been acquired in the case that no significant change in the expression level is found while the dose is reduced and withdrawal from the immunosuppressive agent has been achieved. Examples of the gene marker to be used is a gene-related substance related to a gene selected from the group consisting of IRF1, PSMB9, NOS2A, PIM1, TAP1, and CTSS. The aforementioned tissue or organ is, for example, a heart, a kidney, a liver, etc.

The kit for judging the presence or absence of immunological tolerance according to the present invention includes a primer or an antibody for measuring a change in the expression level of a gene marker in blood, and the gene marker to be used is a gene-related substance related to a gene selected from the group consisting of IRF1, PSMB9, NOS2A, PIM1, TAP1, and CTSS. For example, the present invention may be a kit for judging the presence or absence of immunological tolerance to transplantation of a tissue or an organ, such as a heart, a kidney, a liver, etc.

The method for evaluating the efficacy of an immunosuppressive agent according to the present invention evaluates the efficacy of an immunosuppressive agent by measuring a change in the expression level of a gene marker in blood, which associate with the use of an immunosuppressive agent, and the gene marker to be used is a gene-related substance related to a gene selected from the group consisting of IRF1, PSMB9, NOS2A, PIM1, TAP1, and CTSS. For example, the present invention may be a method for evaluating the efficacy of an immunosuppressive agent on transplantation of a tissue or an organ, such as a heart, a kidney, a liver, etc.

The method for identifying an immunosuppressive agent for a vertebrate requiring administration of an immunosuppressive agent according to the present invention includes the steps of: drawing blood from a plurality of individuals of the vertebrate family and measuring the expression level of a gene marker in the blood, before and after administering an equal amount of different immunosuppressive agents; comparing the expression levels of the gene marker in the blood drawn from the individual between before and after administering the immunosuppressive agent; and identifying the immunosuppressive agent which has decreased the expression level of the gene marker most markedly by the administration of the immunosuppressive agent. The gene marker to be used is a gene-related substance related to a gene selected from the group consisting of TRF1, PSMB9, NOS2A, PIM1, TAP1, and CTSS. For example, the present invention may be a method for identifying an immunosuppressive agent effective for transplantation of a tissue or an organ, such as a heart , a kidney, a liver, etc.

The method for selecting an immunosuppressive agent according to the present invention is to select the most effective immunosuppressive agent for the individual requiring an immunosuppressive agent among a plurality of the immunosuppressive agents by using the calibration curves, constructed for each of the plurality of immunosuppressive agents, which represents a correlation between the amount used or the blood concentration of the immunosuppressive agent and the expression level of a gene marker in a vertebrate individual requiring administration of an immunosuppressive agent. The gene marker to be used is a gene-related substance related to a gene selected from the group consisting of IRF1, PSMB9, NOS2A, PIM1, TAP1, and CTSS. The aforementioned individual is preferably the one that has undergone transplantation of a tissue or an organ, such as a heart, a kidney, a liver, etc.

The method for determining the dose of an immunosuppressive agent according to the present invention is to draw blood from a vertebrate with a disease or rejection which requires administration of the immunosuppressive agent; measure the expression level of a gene marker in the blood; and determine the dose of the immunosuppressive agent in the vertebrate, which is necessary to decrease the expression level of a gene marker in the blood of the vertebrate to a predetermined expression level by using a previously constructed calibration curve which represents a correlation between the amount used or the blood concentration of the immunosuppressive agent and the expression level of the gene marker. The gene marker to be used is a gene-related substance related to a gene selected from the group consisting of IRF1, PSMB9, NOS2A, PIM1, TAP1, and CTSS. The aforementioned individual is preferably the one that has undergone transplantation of a tissue or an organ, such as a heart, a kidney, a liver, etc.

The kit for evaluating the efficacy of an immunosuppressive agent according to the present invention includes a primer or an antibody for measuring a change in the expression level of a gene marker in blood, and the gene marker to be used is a gene-related substance related to a gene selected from the group consisting of IRF1, PSMB9, NOS2A, PIM1, TAP1, and CTSS. The aforementioned immunosuppressive agent may be the one that is used after transplantation of a tissue or an organ, such as a heart, a kidney, a liver, etc.

Further, the method for either screening for an immunosuppressive agent or screening for an immunological tolerance-inducing agent according to the present invention includes the steps of: administering a test substance to a vertebrate in which a tissue or organ transplant causes immunological rejection; drawing blood from the vertebrate; and monitoring the expression level of a gene marker in the blood. The gene marker to be used is a gene-related substance related to a gene selected from the group consisting of IRF1, PSMB9, NOS2A, PIM1, TAP1, and CTSS. The aforementioned tissue or the organ is, for example, a heart, a kidney, a liver, etc.

The method for evaluating the efficacy of an immunosuppressive agent according to the present invention includes measuring the expression level of a gene marker whose expression changes in correlation with the efficacy of an immunosuppressive agent.

The kit for measuring the efficacy of an immunosuppressive agent according to the present invention includes a primer or an antibody for measuring the expression level of a gene marker whose expression changes in correlation with the efficacy of an immunosuppressive agent.

The method for judging the presence or absence of immunological tolerance according to the present invention includes measuring a change in the expression level of a gene marker in blood drawn from a vertebrate into which a tissue or an organ has been transplanted.

The kit for judging the presence or absence of the immunological tolerance according to the present invention includes a primer or an antibody for measuring the expression level of a gene marker in blood drawn from a vertebrate into which a tissue or an organ has been transplanted.

Further, the method for screening for an immunosuppressive agent or an immunological tolerance-inducing agent according to the present invention includes the steps of: administering a test substance to a vertebrate in which a tissue or organ transplant causes immunological rejection; drawing blood from the vertebrate; and monitoring the expression level of a gene marker in the blood.

The method for suppressing rejection according to the present invention includes the steps of measuring the expression level of a gene marker while monitoring the blood concentration of an immunosuppressive agent, and administering the immunosuppressive agent until a predetermined value is reached. The method makes it possible to suppress effectively rejection caused by tissue or organ transplantation.

Further, the method for improving or treating an immunological disease according to the present invention includes the steps of measuring the expression level of a gene marker while monitoring the blood concentration of an immunosuppressive agent, and administering the immunosuppressive agent until a predetermined value is reached. The method makes it possible to effectively improve or treat immunological diseases such as autoimmune diseases, allergic diseases, atopic diseases, rheumatic diseases, pollinosis, etc.

It should be noted that the term "gene marker" as used herein refers to a gene-related substance serving as an indicator for evaluating the state or action of a particular object having a correlation with the expression level of a particular gene. The gene marker encompasses, for example, a gene itself, an mRNA as a transcript, a peptide as a translation product, a protein as an end product of gene expression, etc. The expression level of a gene marker" as used herein refers to, when a gene marker other than a gene is used, the transcription level (when the marker is a transcript etc.) of the gene from which the gene marker is derived, or the amount of expression (when the marker is a polypeptide or a protein, etc.) at the transcription level. A "vertebrate" as used herein refers to a human and a vertebrate, such as a mouse, a rat, etc. other than a human.

### BRIEF DESCRIPTION OF THE DRAWINGS

FIG. 1 shows temporal changes in the expression levels of gene markers (Irf1, Psmb9, Nos2, and Pim1) caused by rejection or administration of an immunosuppressive agent (cyclosporine or tacrolimus) in one example according to the present invention.
FIG. 2 shows temporal changes in the expression levels of the gene markers caused by administration of an immunosuppressive agent in one example according to the present invention.
FIG. 3 shows the results of PD analysis of drugs using the gene markers in an example according to the present invention,
FIG. 4 shows temporal changes in the expression levels of gene markers (TAP1 and CTss) caused by rejection or administration of an immunosuppressive agent (cyclosporine or tacrolimus) in one example according to the present invention.

### BEST MODE FOR CARRYING OUT THE INVENTION

Embodiments of the present invention accomplished based on the above-described findings are hereinafter described in detail by giving Examples. Unless otherwise explained, methods described in standard sets of protocols such as J. Sambrook and E. F. Fritsch & T. Maniatis (Ed.), "Molecular Cloning, a Laboratory Manual (3rd edition), Cold Spring Harbor Press and Cold Spring Harbor, New York (1989); and F. M. Ausubel, R. Brent, R. E. Kingston, D. D. Moore, J. G. Seidman, J. A. Smith, and K. Struhl (Ed.), "Current Protocols in Molecular Biology, " John Wiley & Sons Ltd., and/or their modified/changed methods are used. When using commercial reagent kits and measuring apparatus, unless otherwise explained, protocols attached to them are used.

The object, characteristics, and advantages of the present invention as well as the idea thereof will be apparent to those skilled in the art from the descriptions given herein. It is to be understood that the embodiments and specific examples of the invention described herein below are to be taken as preferred examples of the present invention. These descriptions are only for illustrative and explanatory purposes and are not intended to limit the invention to these embodiments or examples. It is further apparent to those skilled in the art that various changes and modifications may be made based on the descriptions given herein within the intent and scope of the present invention disclosed herein.

### == Usefulness of gene markers ==

The gene marker according to the present invention is a gene-related substance which is related to a gene whose expression changes in correlation with rejection or the efficacy of an immunosuppressive agent. For example, when the gene marker to be used is a gene-related substance related to a gene selected from the group consisting of IRF1, PSMB9, NOS2A, PIM1, TAP1, and CTSS, the expression levels of these gene markers are increased in leukocytes because of rejection and decreased in leukocytes by an immunosuppressive agent. Also, the expression levels of the gene markers have a correlation with the degree of rejection and the dose and blood concentration of an immunosuppressive agent. Therefore, the gene marker according to the present invention is useful as an indicator of rejection or immunological tolerance, or as an indicator of the efficacy of an immunosuppressive agent. It should be noted that the expression level of the gene marker may correlate inversely with rejection or an immunosuppressive agent.

Further, a plurality of the gene markers may be used for comprehensive diagnosis of rejection, as indicators of the efficacy of an immunosuppressive agent, or for judgment of the presence or absence of immunological tolerance. When a plurality of the gene markers are used in these ways, more precise diagnosis of rejection, more strict evaluation of efficacy, and more precise judgment of the presence or absence of immunological tolerance are possible.

Moreover, by using a gene marker in blood, the blood can be used as a sample, thereby making it possible to periodically measure the expression level of the gene marker in a simple and convenient manner. This enables quick diagnosis of rejection in early stages. Early diagnosis is extremely important in that it enables early preventive measures against rejection, thereby minimizing damage to the transplanted tissue or organ caused by rejection. In addition, drawing blood samples is less invasive and less technically demanding than needle biopsy, and thus can be performed in a simple and convenient manner. Likewise, use of blood as samples allows repeated evaluation of efficacy and diagnosis of immunological tolerance according to the present invention in a quick, simple, and convenient manner.

### == Quantification of gene markers ==

In this embodiment, where the gene marker is a gene or a transcript (mRNA) of a gene, the expression level of the gene marker can be measured (quantified) by measuring the amount of mRNA by, for example, the Northern blotting method, dot blotting method, quantitative reverse transcription-polymerase chain reaction (RT-PCR) method, etc. It is preferred to use the quantitative RT-PCR method in that the method enables measurement of the amount of mRNA by using a very small amount of RNA (isolated from leukocytes).

The primer to be used for the quantitative RT-PCR method is not particularly limited, as long as it can specifically detect the gene marker, but an oligonucleotide consisting of 12 to 26 nucleotides is preferred. The nucleotide sequence is determined based on the sequence information of the following genes of the vertebrate to be examined: interferon regulatory factor 1 (IRF1) ; PSMB9 (proteasome (prosome, macropain) subunit, beta type. 9, also known as LMP2 (low molecular mass polypeptide 2)); NOS2A (nitric oxide synthase 2A (inducible, hepatocytes)) ; PIM1 (pim-1 oncogene); TAP1 (transporter 1, ATP-binding cassette, sub-family B (MDR/TAP)); and CTSS (cathepsin S). Then, the primer containing the determined sequence can be prepared with, for example, a DNA synthesis. To be specific, for example, when the animal is a human, the primer can be prepared based on the sequence information deposited as NM002198 (IRF1), NM002800 and NM148954 (PSMB9), NM000625 and NM153292 (NOS2A), NM002648 (PTM1), NM000593 (TAP1) and NM004079 (CTSS). When the animal is a rat, the primer can be prepared based on the sequence information deposited as NM012591 (Irf1), NM012708 (Psmb9), NM012611 (Nos2), NM017034 (Pim1), NM032055 (TAP1), andNM017320 (CTSS).

On the other hand, when the gene marker is a translation product (a polypeptide) or an end product (a protein) of the gene, the expression level of the gene marker can be measured by, for example, the Western blotting method, the ELISA method, etc., which uses a polyclonal antibody, a monoclonal antibody, etc., specific to the gene marker. However, the measurement method is not limited to these; many other techniques, such as radioimmunoassay (RIA), enzyme immunoassay (EIA), can be used. Hereinbelow, measurement of the expression level of a gene marker will be explained by an example of the ELISA method using a polyclonal antibody.

Blood drawn from a vertebrate is homogenized, and then ultrasonicated. After centrifugation, the supernatant is recovered to obtain a crude extract containing proteins. Then, by using an ELISA plate (e.g., 96-well plate) coated with a polyclonal antibody which specifically binds to the gene marker, the crude protein extract is placed in the wells of the plate for reaction with the gene marker, and, subsequently, with the primary antibody and then secondary antibody (e.g., an enzyme-labeled anti-immunoglobulin antibody). After the reactions, the ELISA plate is washed, color is developed with a substrate for the enzyme, the absorbance is measured with a microplate reader, and the expression level of the protein is quantified by using a previously constructed calibration curve. The expression level of a gene marker can be measured in this manner. The enzyme to be used to label the secondary antibody is exemplified by, but not limited to, horseradish peroxidase (HRP), alkaline phosphatase, etc.

### == Method for diagnosing rejection ==

When a gene marker as described above is used, rejection can be diagnosed by, for example, periodically measuring the expression level of the gene marker in blood drawn from a vertebrate into which a tissue or an organ has been transplanted, and comparing the expression level with an expression level of the gene marker in blood drawn before the tissue or organ transplantation. That is, as a result of measuring the expression levels of the gene marker, if the post-transplantation expression level of the marker has significantly changed compared with the pre-transplantation expression level, or the expression level of the gene marker is significantly temporally changing, it is judged that rejection has occurred or is likely to occur. For example, when the gene marker is s a gene-related substance related to a gene selected from the group consisting of IRF1, PSMB9, NOS2A, PIM1, TAP1, and CTSS, if the expression level has increased significantly compared with the pre-transplantation expression level or is significantly temporally increasing , it is judged that rejection has occurred or is likely to occur. On the other hand, if the post-transplantation expression level of the marker has not changed significantly compared with the pre-transplantation expression level, it is judged that rejection has not occurred. A vertebrate to be diagnosed may be a human or a vertebrate other than a human, such as a mouse, a rat, or the like.

### == Method for evaluating the efficacy of an immunosuppressive agent ==

As described above, the expression level of the gene marker according to the present invention is useful not only as an indicator of rejection but also as an indicator in evaluating the efficacy of an immunosuppressive agent. If it becomes possible to evaluate the efficacy of an immunosuppressive agent by measuring the expression level of a gene marker, the mode of administration of an immunosuppressive agent for individual disease can be easily determined based on scientific evidence. For example, it becomes possible to select an immunosuppressive agent and/or mode of its administration effective in a small dose with minimal adverse side effects.

It should be noted that the diseases to be treated with immunosuppressive agents include immunological diseases such as autoimmune diseases, allergic diseases (such as allergic dermatitis, atopic diseases, pollinosis, etc.), and rheumatic diseases besides rejection.

The immunosuppressive agent is not limited to any specific one as long as it is an agent, for example, an agent that suppresses abnormal immunological reactions, such as rejection in the living body after transplantation, or an agent that suppresses immunity in various diseases. Examples of the immunosuppressive agent include steroids (prednisolone, methylprednisolone, etc.), IL-2 antibodies, IL-2 secretion suppressors (rapamycin, etc.), CN suppressors, such as cyclosporine or FK506 (also referred to as tacrolimus or Prograf), antimetabolite agents (mycophenolate mofetil, etc.), cyclophosphamide, OKT3 (also referred to as muromonab-CD3), antilymphocytic globulin (antithymocyte immunoglobulin), anti-CD4 antibodies, anti-TNF-α antibodies, azathioprine, mizoribine, sulfasalazine, 6-mercaptopurine (6-MP), methotrexate, cytoxazone, gusperimus hydrochloride, or combinations of these agents.

### == Determination of the mode of administration of an immunosuppressive agent ==

In a vertebrate individual that has received an immunosuppressive agent after suffering from the aforementioned disease or undergoing a transplantation surgery, by measuring the expression level of a gene marker in blood (leukocytes) of the vertebrate while monitoring the dose of the immunosuppressive agent or the blood concentration of the immunosuppressive agent and, administering the immunosuppressive agent until the expression level is reached to a predetermined value (e.g. until the difference from a normal level decreased to about 20%), it becomes possible to improve or treat the aforementioned disease or to suppress rejection using a minimal dose of the immunosuppressive agent. This enables effective prevention of side effects such as infection associated with overdosing of the immunosuppressive agent. It should be noted that for initial dose of an immunosuppressive agent, an exemplary amount is the minimum dose of the immunosuppressive agent which is determined based on a calibration curve described below.

### == Construction and application of a calibration curve

Constructing a calibration curve representing a correlation between the expression level of a gene marker and the dose or the blood concentration of an immunosuppressive agent in a vertebrate individual which has received the immunosuppressive agent makes it possible to evaluate efficacy of the immunosuppressive agent from various aspects.

For example, it becomes possible to select the most useful immunosuppressive agent for each of the various vertebrates with immunological disease or rejection, by selecting an immunosuppressive agent which brings the expression level of the gene marker to the normal level in a smallest quantity, or by selecting an immunosuppressive agent by which the largest difference occurs between the dose which causes a side effect and the dose which brings the expression level of the gene marker to the normal level, based on calibration curves constructed for a plurality of immunosuppressive agents.

In addition, by using calibration curves which have been constructed as described above for a plurality of modes of administration, the most effective administration mode can be selected. The modes of administration of an immunosuppressive agent include, for example, an injection to an affected area, subcutaneous, intramuscular, intraperitoneal, or intravenous injections, oral administration, parenteral administrations such as applying and pasting, etc.

Also, for vertebrates with immunological diseases such as autoimmune diseases, allergic diseases, atopic disease, rheumatic disease, and pollinosis, or with rejection, which requires administration of an immunosuppressive agent, it becomes possible to establish an optimal dose of the immunosuppressive agent by using the calibration curve as described above. For example, in a particular human or animal patient, by calculating the difference between the expression level of the gene marker before administration of the immunosuppressive agent and a normal expression level of the gene marker, it is possible to determine the dose of an immunosuppressive agent which can decrease the expression level of a gene marker only by a certain percentage.

### == Method for judging the presence or absence of immunological tolerance ==

According to the method for judging the presence or absence of immunological tolerance according to the present invention, it is possible to judge the presence or absence of immunological tolerance by measuring a change in the expression level of a gene marker in blood drawn from a vertebrate into which a tissue or an organ has been transplanted.

One example of the method for judging the presence or absence of immunological tolerance is the method including monitoring the expression level of a gene marker in blood drawn from a vertebrate into which a tissue or an organ has been transplanted, while reducing the dose of the immunosuppressive agent administered to the vertebrate. Namely, as a result of measuring the expression level of a gene marker, if the expression level of the gene marker shows the same change as that found in the case of judgment for the occurrence of rejection while the dose of the immunosuppressive agent is reduced, it is judged that immunological tolerance is not acquired; and if no significant change in the expression level of the gene marker has been found while the dose of the immunosuppressive agent is reduced and withdrawal from the immunosuppressive agent is achieved, it is judged that immunological tolerance is acquired. For example, when the gene marker is a gene related substance related to a gene selected from the group consisting of IRF1, PSMB9, NOS2A, PIM1, TAP1, and CTSS, if the expression level of the gene marker increases while the dose of an immunosuppressive agent is reduced, it is judged that immunological tolerance is not acquired; and if no significant change in the expression level of the gene marker has been found while the dose of the immunosuppressive agent is reduced and withdrawal from the immunosuppressive agent is achieved, it is judged that immunological tolerance is acquired.

As described above, by judging the presence or absence of immunological tolerance, it becomes possible to discontinue administration of an immunosuppressive agent which is intrinsically unnecessary, and thus to avoid the risk of infections associated with long-term administration of the immunosuppressive agent and/or side effects of the immunosuppressive agent. A vertebrate to be diagnosed may be a human or a vertebrate other than a human, such as a mouse, a rat, or the like.

In addition, the method for judging the presence or absence of immunological tolerance according to the present invention can also be used to develop tolerance-inducing methods or to screen for tolerance-inducing agents.

For example, a tissue or an organ is transplanted into a vertebrate, such as a mouse or a rat, and the animal is given a test substance, or the animal is treated by a test method and then its blood is drawn for monitoring the expression level of a gene marker. By performing the aforementioned procedure repeatedly by using multiple test substances or test methods, it is possible to screen for immunosuppressive agents and immunosuppressing methods as well as for tolerance-inducing agents and tolerance-inducing methods.

As an example of the tolerance-inducing agent, T cells can be desensitized by administration of partial peptides of a peptide which serves as an inducer of immunological tolerance, in which case it is possible to screen various partial peptides to select an effective partial peptide. In addition, the a forementioned procedure can be applied to the screening methods for a compound which selectively inhibits the ICOS/B7h pathway and/or the CD28/CTLA4:B7 pathway, which are supplementary signaling pathways. Further, by changing mode of use and/or mode of administration such that, for example, one or more kinds of immunosuppressive agents are used in a vertebrate into which a tissue or an organ has been transplanted, and monitoring the expression level of the gene marker in the blood drawn from the vertebrate, it is possible to develop an effective tolerance-inducing method.

It should be noted that examples of the aforementioned gene markers that can be used include a gene-related substance related to a gene selected from the group consisting of IRF1, PSMB9, NOS2A, PIM1, TAP1 and CTSS. The vertebrate that can be used is exemplified by a vertebrate, such as a mouse, a rat, and the like.

### === Kit ===

The kit according to the present invention is not limited to any specific one as long as it includes a reagent or apparatus for measuring the expression level of the gene marker. Examples of such reagents or apparatus include primers and reagents used for the quantitative RT-PCR methods, antibodies used for the Western blotting and/or the ELISA method. In addition, the kit may include a reagent for isolating leukocytic RNA from blood, probes for hybridization for gene markers, anti-immunoglobulin secondary antibodies labeled with an enzyme, fluorescent substances, radioactive substances, etc., and a substrate for the enzyme. The enzymes that can be used include horseradish peroxidase (HRP), alkaline phosphatase, etc., the fluorescent substances that can be used include Cy2, FluorX, Cy3, Cy3.5, Cy5, Cy5.5, Cy7, FITC (fluorescein isothiocyanate), rhodamine, etc, and the radioactive substance that can be used include ³H, ³²P, ³⁵S, ¹²¹I, ¹²⁵I, etc.

The aforementioned primers include a primer directed to one or more genes selected from the group consisting of IRF1, PSMB9, NOS2A, PIM1, TAP1, and CTSS. The aforementioned antibodies that can be used include polyclonal and monoclonal antibodies which react specifically to a gene marker (polypeptides or proteins) related to a gene such as IRF1, PSMB9, NOS2A, PIM1, TAP1, and CTSS. It should be noted that the kit according to the present invention may include one or more antibodies.

### EXAMPLES

Hereinafter, the present invention will be explained in more detail with reference to Examples and drawings.

### [EXAMPLE 1]

By using Lewis rats as donors and isogenic (Lewis) rats as recipients, heart transplantation was performed (non-rejection group; n = 3) . In addition, by using ACI rats as donors and allogeneic (Lewis) rats as recipients, heart transplantation was performed. Three (immunosuppressive agent-administered group; n = 3) out of the six allogeneic recipients received a subcutaneous injection of 5 mg/kg BW of cyclosporine (manufactured by Novartis Pharmaceuticals Corporation; dissolved in saline) on the day of transplantation (day 0), followed by daily subcutaneous injections of cyclosporine at the same dose. The remaining three rats did not receive any immunosuppressive agent (rejection group; n=3) . The Lewis and ACI rats used were 8 to 10-week-oldmales and weighed between 200 and 300 g.

On post-transplantation day 4 (in the rejection group, the cessation of the heartbeat of the transplanted heart had not been confirmed yet), laparotomy was performed on three rats in each of the non-rejection group, rejection group, and immunosuppressive agent-administered group; and peripheral blood (about 5 ml) was drawn from the inferior vena cava. Then, mRNA was extracted with the QIAamp RNA Blood Mini kit (manufactured by QIAGEN Inc.) and DNA was removed by DNase treatment. By using 20 µg of the mRNAs obtained from the peripheral blood, cDNAs labeled with Cy5 dye were obtained with the LabelStar Array kit (manufactured by QIAGEN Inc.). These cDNAs were hybridized with Atlas Glass Microarray Rat 1.0 (manufactured by BD Bio sciences Clontech). The microarrays were scanned and the data were analyzed with an Axon GenePix 4000A scanner (manufactured by Axon Instruments) by using GenePix Pro 3.0 Microarray analysis software (manufactured by Axon Instruments) (with global normalization). As a result, the genes (Irf1, Psmb9, Nos2, Pim1, Tap1, and Ctss) whose expression levels had been markedly increased in the rejection group, compared with the non-rejection group or the immunosuppressive agent-administered group, were selected.

To precisely examine changes in the expression level of each of the genes selected here, their mRNAs were quantified. For Irf1, Psmb9, Nos2, and Pim1, the mRNA was quantified by using 25 ng of extracted mRNA together with each primer and probe shown in Table 1, synthesizing cDNA with TaqMan Reverse Transcription Reagents (manufactured by Applied Biosystems) under the conditions shown in Table 1, on an ABI PRISM 7900HT Detection System (manufactured by Applied Biosystems), and using β-actin as an internal control. For Tap1 and Ctss, mRNA was quantified by using 25 ng of extracted mRNA together with each primer and probe (final concentrations: 900 nM each primer, 250 nM TaqMan probe; TaqMan Gene Expression Assays Rn00709612_m1 (Tap1) or Rn00569036_m1 (Ctss), Applied Biosystems), synthesizing cDNA with TaqMan Reverse Transcription Reagents (manufactured by Applied Biosystems) on an ABI PRISM 7900HT Detection System (manufactured by Applied Biosystems), and using β-actin as an internal control.

**TABLE 1**

| Name of gene (Accession No. of mRNA sequence) | Primer β-Actin: final concentration 200 nM Other genes: final concentration 900 nM | Probe β-Actin: final concentration 100 nM Other genes: final concentration 250 nM | Reaction condition |
|---|---|---|---|
| β-Actin (NM031144) | GCCGTCTTCCCCTCCAT (SEQ ID NO: 1) AGGAGTCCTTCTGACCCATACC (SEQ ID NO: 2) | (VIC)-CCATCACACCCTGGTGCC-(MGB) (SEQ ID NO: 11) | |
| Irf1 (NM012691) | CACCACTGATCTGTACAACTTGCA (SEQ ID NO: 3) CACTCAGACTGTTCAAAGAGCTTCA (SEQ ID NO: 4) | (FAM)-ACCTCTGAAGCTGCAACA-(MGB) (SEQ ID NO: 12) | 50°C for 2 min, 95°C for 10 min, for initial heat denaturation, followed by a PCR reaction with 40 cycles of: (95°C 15 s, 60°C 60 s) per cycle |
| Psmb9 (NM012708) | AGAAGTCCACACCGGGACAAC (SEQ ID NO: 5) TGTCAAACACGCGGTTCACTA (SEQ ID NO: 6) | (FAM)-GGCTGCTCCCGCTGACACTCG-(TAMRA) (SEQ ID NO: 13) | |
| Pim1 (NM017034) | GGTCTACTCGGGCATCCG (SEQ ID NO: 7) GGTCCTTCTCCACGTGCTT (SEQ ID NO: 8) | (FAM)-ATGGCCACCGCAAGT-(MGB) (SEQ ID NO: 14) | |
| Nos2 (NM012611) | CTGGTGGTGACAAGCACATTTG (SEQ ID NO: 9) GTATGCCCGAGTTCTTTCATCATG (SEQ ID NO: 10) | (FAM)-CTTCAGAGTCTGCCCATTGC-(MGB) (SEQ ID NO: 15) | |

As a result, the Irf1, Psmb9, Nos2, Pim1, Tap1, and Ctss genes, whose expression levels had been increased by 1.5-fold or more because of rejection caused by the heart transplantation from allogeneic rats and whose expression levels had been decreased by 1.5-f old or more because of administration of an immunosuppressive agent, were identified.

### [EXAMPLE 2]

To examine whether the genes identified in Example 1 can serves as indicators of rejection or the efficacy of an immunosuppressive agent, 200 µl of blood was drawn from the caudal vein of individual mouse in the non-rejection group, rejection group, and immunosuppressive agent-administered group (the cyclosporine-administered group received a subcutaneous injection of 5 mg/kg BW of cyclosporine on the day of transplantation, followed by daily subcutaneous injections; the tacrolimus-administered group received a subcutaneous injection of 1.28 mg/kg BW of cyclosporine on the day of transplantation, followed by daily subcutaneous injections), starting from the day before transplantation and daily until post-transplantation day 3. On post-transplantation day 4, laparotomy was performed on individual rats, 200 µl of blood was drawn from the inferior vena cava, and expression levels of gene markers (Irf1 (n = 5), Psmb9 (n = 5), Nos2 (n = 3) or Pim1 (n = 3); for the tacrolimus-administered group only, all in n = 3) were measured. The expression levels of the gene markers were measured by quantifying mRNAs, which had been extracted from blood in the same manner as described in Example 1. In this quantitative analysis, post-transplantation expression levels of the gene markers were relatively quantified by using the pre-transplantation expression levels of the gene markers as the reference (as "1").

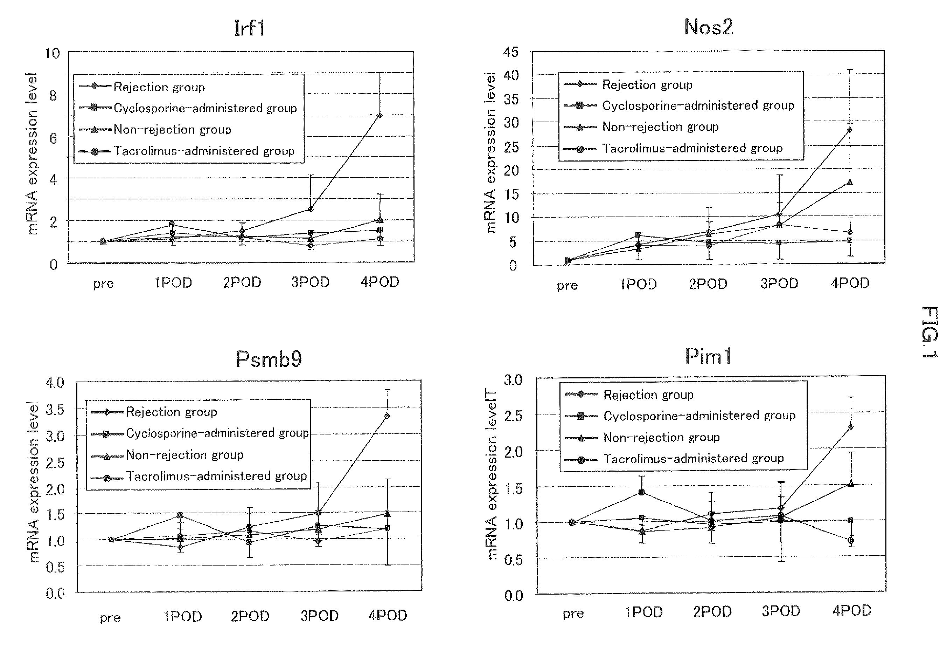

As shown in FIG. 1, in the rejection group the expression levels of the gene markers were increased significantly compared with the non-rejection group, whereas in the immunosuppressive agent-administered group, the expression levels of the gene markers were suppressed compared with the rejection group, to the level similar to or lower than that of the non-rejection group.

### [EXAMPLE 3]

Next, to ascertain whether the expression levels of the gene markers change depending on the dose of an immunosuppressive agent, on the day before transplantation, on post-transplantation day 2, and on post-transplantation day 4, 200 µl of blood was drawn from the caudal vein of rats which had received a subcutaneous injection of 1 mg, 2 mg, or 3 mg/kg BW of cyclosporine on the day of transplantation, followed by daily subcutaneous injections. Then expression levels of gene markers (Irf1, Psmb9, Nos2, or Pim1) were measured. The expression levels of the gene markers were measured by quantifying their mRNAs, which had been extracted from blood in the same manner as described in Example 1.

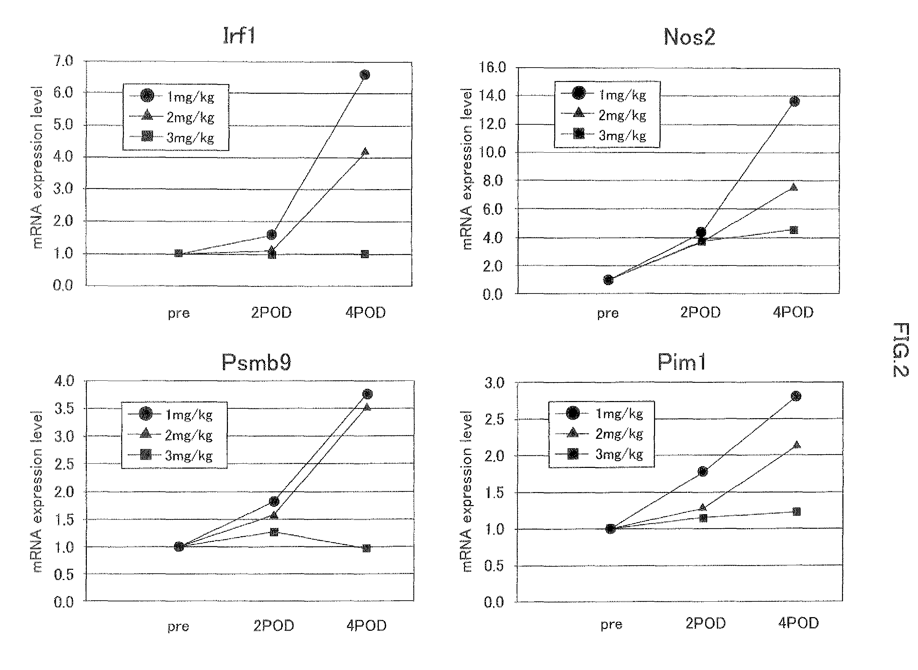

As shown in FIG. 2, it was confirmed that for each gene marker the expression level was decreased with increasing dose of cyclosporine. This indicates that the dose of cyclosporine correlates with the expression level of the gene marker (Irf1, Psmb9, Nos2, or Pim1). This correlation does not reflect a correlation between the efficacy of a particular agent and the expression level of the gene marker, but a correlation between the degree of rejection and the expression level of the gene marker, suggesting that the agent to be used is not particularly limited to cyclosporine.

### [EXAMPLE 4]

Next, to examine whether the expression levels of the gene markers correlate with the blood concentration of an agent, appropriate doses of cyclosporine were administered to rejection model rats and pharmacodynamics (PD) analysis was performed by using expression levels of the gene markers as indicators. The expression levels of the gene markers ware measured by quantifying their mRNA, which had been obtained from blood in the same manner as described in Example 1. The blood concentration of the agent was measured by the fluorescence polarization immunoassay (FPIA) . As a measuring apparatus and a measuring agent, TDXFLX (Abbott Japan Co., Ltd.) and Cyclosporine-SP-Dynapack (Abbott Japan Co., Ltd.) were used, respectively.

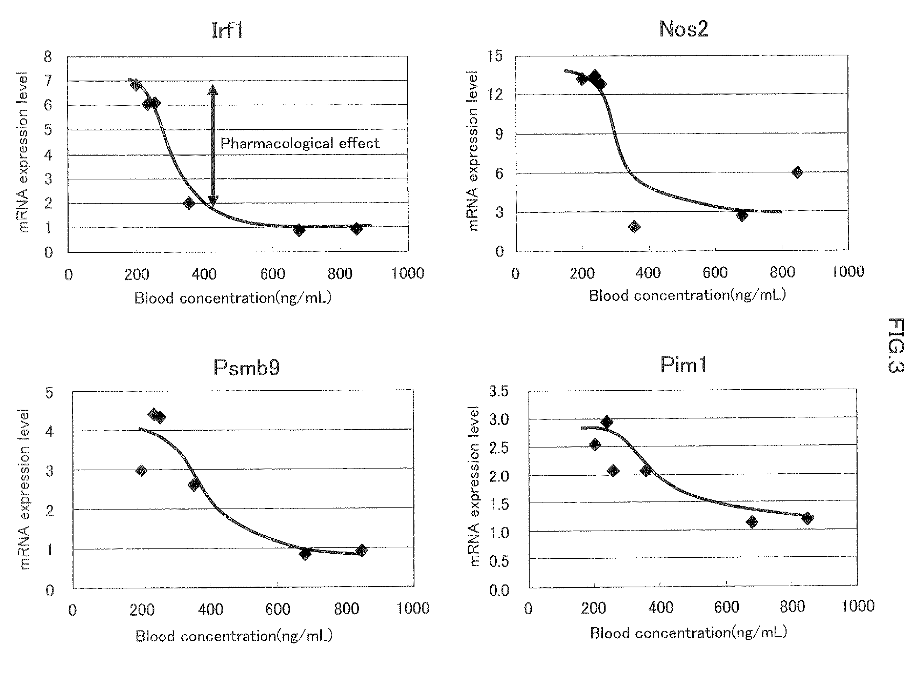

As shown in FIG. 3, until the blood concentration of the agent reached 200 ng/mL, the expression levels of none of the gene markers (Irf1, Psmb9, Nos2, or Pim1) were substantially decreased. After the concentration exceeded 200 ng/mL, the expression levels of the gene markers were gradually decreased. When the concentration exceeded 400 g/ml, the expression levels of the mRNA decreased to the expression level before the organ transplantation. This indicates that the blood concentration of the agent correlates with the expression levels of the gene markers.

The Examples described so far revealed that the gene markers (Irf1, Psmb9, Nos2, and Pim1) are useful as indicators for diagnosis of rejection, judgment of the presence or absence of immunological tolerance, prediction of potential rejection or immunological tolerance, and/or the efficacy of an immunosuppressive agent.

### [EXAMPLE 5]

Next, on the assumption that genes such as Tap1 and Ctss, which were identified in Example 1, might also serve as indicators of rejection or the efficacy of an immunosuppressive agent, 200 µl of blood was drawn in the same manner as described in Example 2, from the caudal vein of individual mouse in the non-rejection group, the rejection group, and the immunosuppressive agent-administered group (the cyclosporine-administered group and the tacrolimus-administered group), starting from the day before transplantation and daily until post-transplantation day 3. On post-transplantation day 4, laparotomy was performed on individual rats, 200 µl of blood was drawn from the inferior vena cava, and expression levels of the gene markers (Tapl (n = 2) or Ctss (n = 2); for the tacrolimus-administered group only, both inn =3.) were measured. The expression levels of the gene markers were measured by quantifying their mRNA, which had been obtained from blood in the same manner as described in Example 1. In this quantitative analysis, post-transplantation expression levels of the gene markers were relatively quantified by using the pre-transplantation expression levels of the gene markers as the reference (as "1").

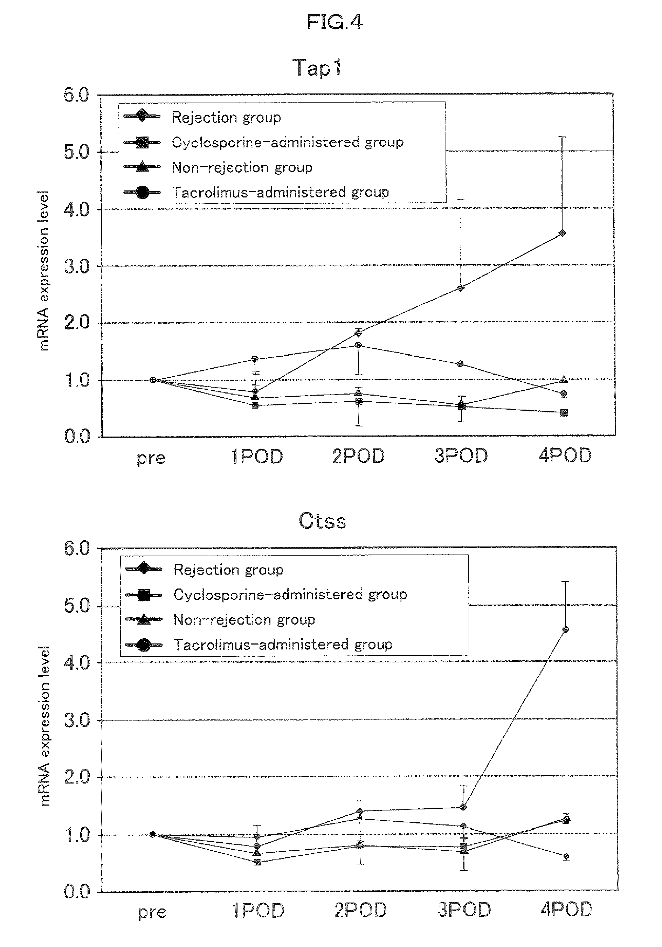

As shown in FIG. 4, it was revealed that in the rejection group, the expression levels of the gene markers such as Tap1 and Ctss, like the gene markers such as Irf1, Psmb9, Nos2, and Pim1, were increased significantly compared with the non-rejection group, whereas in the immunosuppressive agent-administered groups (the cyclosporine-administered group and the tacrolimus-administered group), the expression levels of the gene markers were suppressed compared with the rejection group, to the level similar to or lower than that of the non-rejection group. These results indicate that there are correlations between the gene markers such as Tap1 and Ctss and the dose of cyclosporine or tacrolimus. Also, these gene markers were suggested to be useful for diagnosis of rejection, judgment of the presence or absence of immunological tolerance, prediction of potential rejection or immunological tolerance, and/or the efficacy of an immunosuppressive agent.

### INDUSTRIAL APPLICABILITY

In accordance with the present invention, gene markers which enable diagnosis of rejection, evaluation of the efficacy of immunosuppressive agents, and judgment of the presence or absence of immunological tolerance; methods that can be quickly and conveniently performed by using the gene marker as an indicator for diagnosing rejection, evaluating the efficacy of immunosuppressive agents, identifying immunosuppressive agents, selecting immunosuppressive agents, determining the doses of immunosuppressive agents, and judging the presence or absence of immunological tolerance; kits; and methods for screening for immunosuppressive agents and immunological tolerance-inducing agents, can be provided.

## Claims

1. A gene marker which serves as an indicator of rejection, the gene marker being a gene-related substance related to a gene selected from the group consisting of IRF1, PSMB9, NOS2A, and PIM1.

2. A gene marker which serves as an indicator of rejection, the gene marker being a gene-related substance related to the TAP1 or CTSS gene.

3. The gene marker of claim 1 or 2, wherein the gene marker serves as an indicator of rejection due to heart transplantation.

4. A gene marker which serves as an indicator of immunological tolerance, the gene marker being a gene-related substance related to a gene selected from the group consisting of IRF1, PSMB9, NOS2A, and PIM1,

5. A gene marker which serves as an indicator of immunological tolerance, the gene marker being a gene-related substance related to the TAP1 or CTSS gene.

6. The gene marker of claim 4 or 5, wherein the gene marker serves as an indicator of immunological tolerance to heart transplantation.

7. A gene marker which serves as an indicator of the efficacy of an immunosuppressive agent, the gene marker being a gene-related substance related to a gene selected from the group consisting of TRF1, PSMB9, NOS2A, and PIM1.

8. A gene marker which serves as an indicator of the efficacy of an immunosuppressive agent, the gene marker being a gene-related substance related to the TAP1 or CTSS gene.

9. The gene marker of claim 7 or 8, wherein the gene marker serves as an indicator of the efficacy of an immunosuppressive agent on heart transplantation.

10. A method for diagnosing rejection by measuring a change in the expression level of a gene marker in blood drawn from a vertebrate other than a human, the gene marker being a gene-related substance related to a gene selected from the group consisting of IRF1, PSMB9, NOS2A, and PIM1.

11. A method for diagnosing rejection by measuring a change in the expression level of a gene marker in blood drawn from a vertebrate other than a human, the gene marker being a gene-related substance related to the TAP1 or CTSS gene.

12. The method for diagnosing rejection of claim 10 or 11, wherein the method is for diagnosing rejection due to heart transplantation.

13. A kit for diagnosing rejection, comprising a primer or an antibody for measuring a change in the expression level of a gene marker in blood, the gene marker being a gene-related substance related to a gene selected from the group consisting of IRF1, PSMB9, NOS2A, and PIM1.

14. A kit for diagnosing rejection, comprising a primer or an antibody for measuring a change in the expression level of a gene marker in blood, the gene marker being a gene-related substance related to the TAP1 or CTSS gene.

15. The kit for diagnosing rejection of claim 13 or 14, wherein the kit is for diagnosing rejection due to heart transplantation.

16. A method for judging the presence or absence of immunological tolerance by measuring a change in the expression level of a gene marker in blood drawn from a vertebrate other than a human into which a tissue or an organ has been transplanted, the gene marker being a gene-related substance related to a gene selected from the group consisting of IRF1, PSMB9, NOS2A, and PIM1.

17. A method for judging the presence or absence of immunological tolerance by measuring a change in the expression level of a gene marker in blood drawn from a vertebrate other than a human into which a tissue or an organ has been transplanted, the gene marker being a gene-related substance related to the TAP1 or CTSS gene.

18. The method for judging the presence or absence of immunological tolerance of claim 16 or 17, wherein the tissue or the organ is a heart.

19. A method for judging the presence or absence of immunological tolerance, comprising the steps of: monitoring the expression level of a gene marker in blood drawn from a vertebrate other than a human into which a tissue or an organ has been transplanted while reducing the dose of an immunosuppressive agent administered to the vertebrate other than a human after the transplantation; and judging that the immunological tolerance is acquired in the case that no significant change in the expression level is found while the dose is reduced and withdrawal from the immunosuppressive agent is achieved.

20. The method for judging the presence or absence of immunological tolerance of claim 19, wherein the gene marker is a gene-related substance related to a gene selected from the group consisting of IRF1, PSMB9, NOS2A, and PIM1.

21. The method for judging the presence or absence of immunological tolerance of claim 19, wherein the gene marker is a gene-related substance related to the TAP1 or CTSS gene.

22. The method for judging the presence or absence of immunological tolerance of anyone of claims 19 to 21, wherein the tissue or the organ is a heart.

23. A kit for judging the presence or absence of immunological tolerance, comprising a primer or an antibody for measuring a change in the expression level of a gene marker in blood, the gene marker being a gene-related substance related to a gene selected from the group consisting of IRF1, PSMB9, NOS2A, and PIM1.

24. A kit for judging the presence or absence of immunological tolerance, comprising a primer or an antibody for measuring a change in the expression level of a gene marker in blood, the gene marker being a gene-related substance related to the TAP1 or CTSS gene.

25. The kit for judging the presence or absence of immunological tolerance of claim 23 or 24, wherein the kit is for judging the presence or absence of immunological tolerance to heart transplantation.

26. A method for evaluating the efficacy of an immunosuppressive agent, comprising the step of measuring a change in the expression level of a gene marker in blood associated with a use of the immunosuppressive agent, the gene marker being a gene-related substance related to a gene selected from the group consisting of IRF1, PSMB9, NOS2A and PIM1.

27. A method for evaluating the efficacy of an immunosuppressive agent, comprising the step of measuring a change in the expression level of a gene marker in blood associated with a use of the immunosuppressive agent, the gene marker being a gene-related substance related to the TAP1 or CTSS gene.

28. The method for evaluating the efficacy of an immunosuppressive agent of claim 26 or 27, wherein the method is for evaluating the efficacy of an immunosuppressive agent on heart transplantation.

29. A method for identifying an effective immunosuppressive agent for a vertebrate requiring administration of an immunosuppressive agent, comprising the steps of:
drawing blood from a plurality of individuals of the vertebrate family, before and after administering an equal amount of different immunosuppressive agents;
measuring the expression level of a gene marker in the blood, the gene marker being a gene-related substance related to a gene selected from the group consisting of IRF1, PSMB9, NOS2A, and PIM1;
comparing the expression levels of the gene marker in the blood drawn from the individual between before and after administering the immunosuppressive agent; and
identifying the immunosuppressive agent which has decreased the expression level of the gene marker most markedly by the administration of the immunosuppressive agent.

30. A method for identifying an effective immunosuppressive agent for a vertebrate requiring administration of an immunosuppressive agent, comprising the steps of:
drawing blood from a plurality of individuals of the vertebrate family, before and after administering an equal amount of different immunosuppressive agents;
measuring the expression level of a gene marker in the blood, the gene marker being a gene-related substance related to the TAP1 or CTSS gene;
comparing the expression levels of the gene marker in the blood drawn from the individual between before and after administering the immunosuppressive agent; and
identifying the immunosuppressive agent which has decreased the expression level of the gene marker most markedly by the administration of the immunosuppressive agent.

31. The method for identifying an effective immunosuppressive agent of claim 29 or 30, wherein the immunosuppressive agent is for heart transplantation.

32. A method of selecting an immunosuppressive agent, comprising the steps of:
constructing a calibration curve, for each of a plurality of immunosuppressive agents, which represents a correlation between the amount used or the blood concentration of one of the immunosuppressive agents and the expression level of a gene marker in a vertebrate individual requiring administration of an immunosuppressive agent, the gene marker being a gene-related substance related to a gene selected from the group consisting of IRF1, PSMB9, NOS2A, and PIM1, and
selecting the most effective immunosuppressive agent for the individual requiring an immunosuppressive agent among the plurality of the immunosuppressive agents by using the calibration curves.

33. A method of selecting an immunosuppressive agent, comprising the steps of:
constructing a calibration curve, for each of a plurality of immunosuppressive agents, which represents a correlation between the amount used or the blood concentration of one of the immunosuppressive agents and the expression level of a gene marker in a vertebrate individual requiring administration of an immunosuppressive agent, the gene marker being a gene-related substance related to the TAP1 or CTSS gene, and
selecting the most effective immunosuppressive agent for the individual requiring an immunosuppressive agent among the plurality of the immunosuppressive agents by using the calibration curves.

34. The method for selecting an immunosuppressive agent of claim 32 or 33, wherein the individual has received a heart transplant.

35. A method for determining the dose of an immunosuppressive agent, comprising the steps of:
drawing blood from a vertebrate with a disease or rejection which requires administration of the immunosuppressive agent;
measuring the expression level of a gene marker in the blood, the gene marker being a gene-related substance related to a gene selected from the group consisting of IRF1, PSMB9, NOS2A, and PIML; and
determining the dose of the immunosuppressive agent necessary to decrease the expression level of the gene marker in the blood of the vertebrate to a predetermined expression level by using a previously constructed calibration curve which represents a correlation between the amount used or the blood concentration of the immunosuppressive agent and the expression level of the gene marker.

36. A method for determining the dose of an immunosuppressive agent, comprising the steps of:
collecting blood from a vertebrate with a disease or rejection which requires administration of the immunosuppressive agent;
measuring the expression level of a gene marker in the blood, the gene marker being a gene-related substance related to the TAP1 or CTSS gene; and
determining the dose of the immunosuppressive agent necessary to decrease the expression level of the gene marker in the blood of the vertebrate to a predetermined expression level by using a previously constructed calibration curve which represents a correlation between the amount used or the blood concentration of the immunosuppressive agent and the expression level of the gene marker.

37. The method for determining the dose of an immunosuppressive agent of claim 35 or 36, wherein the individual has received a heart transplant.

38. A kit for evaluating the efficacy of an immunosuppressive agent, comprising a primer or an antibody for measuring a change, associated with a use of the immunosuppressive agent, in the expression level of a gene marker in blood, the gene marker being a gene-related substance related to a gene selected from the group consisting of IRF1, PSMB9, NOS2A, and PIM1.

39. A kit for evaluating the efficacy of an immunosuppressive agent, comprising a primer or an antibody for measuring a change associated with a use of the immunosuppressive agent, in the expression level of a gene marker in blood, the gene marker being a gene-related substance related to the TAP1 or CTSS gene.

40. A kit for evaluating the efficacy of an immunosuppressive agent of claim 38 or 39, wherein the immunosuppressive agent is used after heart transplantation.

41. A method for screening for an immunosuppressive agent or an immunological tolerance-inducing agent, comprising the steps of:
administering a test substance to a vertebrate in which a tissue or an organ transplant causes immunological rejection;
collecting blood from the vertebrate; and
monitoring the expression level of a gene marker in the blood, the gene marker being a gene-related substance related to a gene selected from the group consisting of IRF1, PSMB9, NOS2A, and PIM1.

42. A method for screening for an immunosuppressive agent or an immunological tolerance-inducing agent, comprising the steps of:
administering a test substance to a vertebrate in which a tissue or an organ transplant causes immunological rejection;
collecting blood from the vertebrate; and
monitoring the expression level of a gene marker in the blood, the gene marker being a gene-related substance related to the TAP1 or CTSS gene.

43. The method for screening of claim 41 or 42, wherein the tissue or the organ is a heart.

44. A method for evaluating the efficacy of an immunosuppressive agent, comprising the step of measuring the expression level of a gene marker whose expression changes in correlation with the efficacy of the immunosuppressive agent.

45. A kit for measuring the efficacy of an immunosuppressive agent, comprising a primer or an antibody for measuring the expression level of a gene marker whose expression changes in correlation with the efficacy of the immunosuppressive agent.

46. A method for judging the presence or absence of immunological tolerance, comprising the step of measuring a change in the expression level of a gene marker in blood drawn from a vertebrate other than a human into which a tissue or an organ has been transplanted.

47. A kit for judging the presence or absence of immunological tolerance, comprising a primer or an antibody for measuring the expression level of a gene marker in blood drawn from a vertebrate into which a tissue or an organ has been transplanted.

48. A method for screening for an immunosuppressive agent or an immunological tolerance-inducing agent, comprising the steps of:
administering a test substance to a vertebrate in which a tissue or an organ transplant causes immunological rejection;
drawing blood from the vertebrate; and
monitoring the expression level of a gene marker in the blood.
